# EUROPEAN PATENT SPECIFICATION

(11) **EP 3 791 818 B1**
(45) Date of publication and mention of the grant of the patent: **30.04.2025**
(21) Application number: 20195648.9
(22) Date of filing: 11.09.2020
(51) Int. Cl.: A61B 18/14

(54) **SEALS AND REINFORCEMENT FOR IRRIGATED ELECTROPHYSIOLOGY BALLOON CATHETER WITH FLEXIBLE-CIRCUIT ELECTRODES**
DICHTUNGEN UND VERSTÄRKUNG FÜR ELEKTROPHYSIOLOGIE-BALLONKATHETER MIT FLEXIBLEN SCHALTUNGSELEKTRODEN
JOINTS ET RENFORCEMENT POUR CATHÉTER À BALLONNET D'ÉLECTROPHYSIOLOGIE IRRIGUÉ AU MOYEN D'ÉLECTRODES À CIRCUIT FLEXIBLE

(30) Priority: 13.09.2019 US 201962900129 P; 21.08.2020 US 202017000154
(43) Date of publication of application: 17.03.2021
(73) Proprietor: Biosense Webster (Israel) Ltd., Yokneam 2066717 (IL)
(72) Inventor: NGO-CHU, Don Q., Pasadena, CA California 91107 (US); BEECKLER, Christopher T., Irvine, CA California 92618 (US); PHAM, Cuong, Westminster, CA California 92683 (US); HERRERA, Kevin J., Irvine, CA California 92618 (US); KEYES, Joseph T., Irvine, CA California 92618 (US)
(74) Representative: Carpmaels & Ransford LLP

(56) References cited:
- EP-A2- 3 332 727
- WO-A1-2018/106569
- WO-A2-2011/143468
- US-A1- 2015 265 812

## Description

### FIELD

The subject matter disclosed herein relates to ablation systems, particularly those that include a catheter capable of ablating cardiac tissue and a graphical user interface system to assist in performing the ablation.

### BACKGROUND

Ablation of cardiac tissue has been used to treat cardiac arrhythmias. Ablative energies are typically provided to cardiac tissue by a tip portion, which can deliver ablative energy alongside the tissue to be ablated. Some of these catheters administer ablative energy from various electrodes disposed on or incorporated into three-dimensional structures, e.g., wire baskets and balloons.

### SUMMARY OF THE DISCLOSURE

An irrigated electrophysiology balloon catheter with flexible-circuit electrodes is disclosed. The catheter may include an outer tubular shaft having an outer surface, a first lumen disposed therethrough, and a second lumen disposed therethrough. It may also include an inner tubular shaft having an outer surface disposed in the first lumen of the outer tubular shaft and having a distal portion that extends out of a distal portion of the outer tubular shaft. A catheter balloon may also be provided. The catheter balloon may include a membrane having a first end and a second end, the first end connected to the outer surface of the outer tubular shaft about the distal portion of the outer tubular shaft, and the second end connected to the outer surface of the distal portion of the inner tubular shaft. As such, a distal segment of the inner tubular shaft may be disposed in the balloon.

To help prevent fluids, e.g., irrigation fluid or air, from passing between a space between the inner tubular shaft and the outer tubular shaft, a first seal and a second seal may also be provided about the locations where the inner tubular shaft emerges from the outer tubular shaft. The materials and dimensions of these seals are instrumental in ensuring that the seals are sufficiently robust such that they do not fail during use of the catheter.

The flexible-circuit electrodes may include substrates disposed on the balloon. Delamination of these substrates may be prevented by use of a reinforcement component, e.g., a Liquid Crystal Polymer (LCP) or Ultra High Molecular Weight Polyethylene (UHMWPE) yarn, that extends the length of the balloon. WO 2018/106569 A1 describes an ablation balloon and catheter shaft that deflects to conform to a shape of a target pulmonary vein receiving ablation therapy for a cardiac arrhythmia. EP 3332727 A2 describes an irrigated balloon catheter with support spines and variable shape. WO 2011/143468 A2 describes a low profile electrode assembly. US 2015/265812 A1 describes a balloon catheter with a reduced distal length.

### BRIEF DESCRIPTION OF THE DRAWINGS

While the specification concludes with claims, which particularly point out and distinctly claim the subject matter described herein, it is believed the subject matter will be better understood from the following description of certain examples taken in conjunction with the accompanying drawings, in which like reference numerals identify the same elements and in which:
Figure 1 is a schematic illustration of an invasive medical procedure;
Figure 2 is a top schematic view of a catheter with a balloon in an expanded state;
Figure 3 is a perspective schematic view of the balloon of Figure 2, along with a lasso catheter;
Figure 4 is a side schematic view of a distal end of the catheter of Figure 2 deployed in the region of a pulmonary vein and its ostium;
Figure 5 depicts a cross-section view of a solid model of the balloon of Figure 2 that includes a first seal;
Figure 6 depicts a cross-section drawing of the first seal;
Figure 7 depicts a cross-section view of a solid model of a portion of a handle of the catheter of Figure 2 that includes a second seal.
Figure 8 depicts a cross-section view of a solid model of the second seal;
Figure 9 depicts a perspective view of a solid model of the second seal;
Figure 10 depicts a cross-section drawing of the second seal; and
Figure 11 depicts an image of a substrate of the balloon of Figure 2 having a reinforcement component.

### MODES OF CARRYING OUT THE INVENTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g. "about 90%" may refer to the range of values from 81% to 99%. In addition, as used herein, the terms "patient," "host," "user," and "subject" refer to any human or animal subject and are not intended to limit the systems or methods to human use, although use of the subject invention in a human patient represents a preferred embodiment. Where values are provided in inches, 1 inch = 2.54 cm.

### System Description

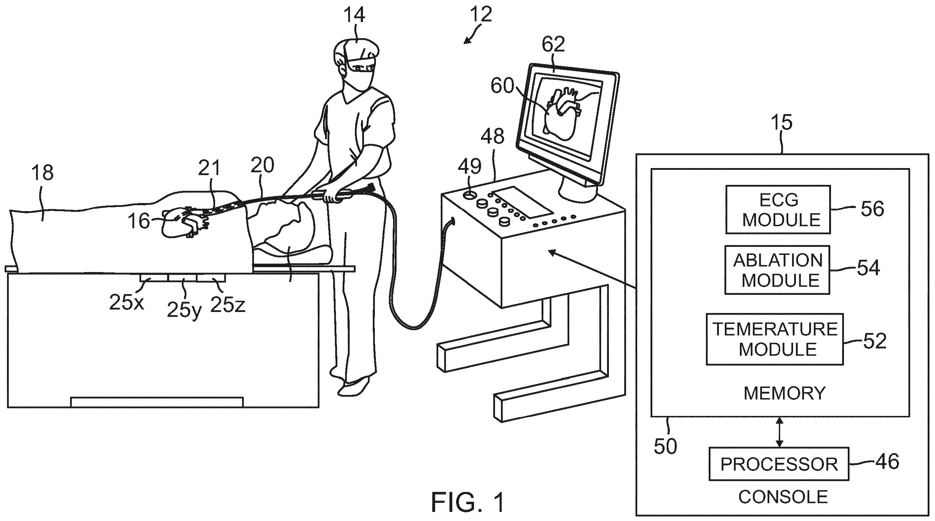

Figure 1 is a schematic illustration of an invasive medical procedure using apparatus 12, according to an embodiment. The procedure is performed by a medical professional 14, and, by way of example, the procedure in the description hereinbelow is assumed to comprise ablation of a portion of a myocardium 16 of the heart of a human patient 18. However, it is understood that embodiments disclosed herein are not merely applicable to this specific procedure and may include substantially any procedure on biological tissue or on non-biological materials.

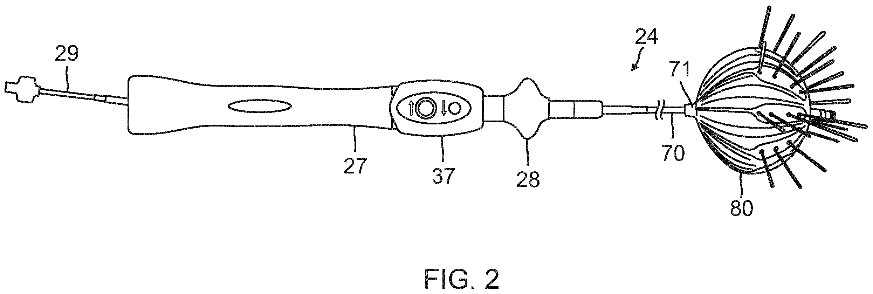

To perform the ablation, medical professional 14 inserts a probe 20 into a sheath 21 that has been pre-positioned in a lumen of the patient. Sheath 21 is positioned so that a distal end of probe 20 enters the heart of the patient. A diagnostic/therapeutic catheter 24 (e.g., a balloon catheter), which is described in more detail below with reference to Figure 2, is deployed through a lumen of the probe 20, and exits from a distal end of the probe 20.

As shown in Figure 1, apparatus 12 is controlled by a system processor 46, which is in an operating console 48 of the apparatus, also shown schematically at reference numeral 15. Console 48 comprises controls 49 and screen 62, which may be used by medical professional 14 to communicate with the processor. As such, screen 62 may comprise a touch screen and controls 49 may comprise, e.g., a mouse or trackball. During the procedure, processor 46 typically tracks a location and an orientation of the distal end of the probe 20, using any method known in the art. For example, processor 46 may use a magnetic tracking method, wherein magnetic transmitters 25X, 25Y and 25Z external to the patient 18 generate signals in coils positioned in the distal end of the probe 20. The CARTO^{®} system (available from Biosense Webster, Inc. of Irvine, California) uses such a tracking method.

The software for the processor 46 may be downloaded to the processor in electronic form, over a network, for example. Alternatively, or additionally, the software may be provided on non-transitory tangible media, such as optical, magnetic, or electronic storage media. The tracking of the distal end of probe 20 may be displayed on a three-dimensional representation 60 of the heart of the patient 18 on screen 62. However, it may be displayed two-dimensionally, e.g., by fluoroscopy or MRI.

To operate apparatus 12, processor 46 communicates with a memory 50, which has many modules used by the processor to operate the apparatus. Thus, the memory 50 comprises a temperature module 52, an ablation module 54, and an electrocardiograph (ECG) module 56. The memory 50 typically comprises other modules, such as a force module for measuring the force on the distal end of probe 20, a tracking module for operating the tracking method used by the processor 46, and an irrigation module 53 connected to a pump allowing the processor to control the pump, and thus irrigation provided to the catheter. For simplicity, such other modules are not illustrated in Figure 1. The modules may comprise hardware as well as software elements. For example, module 54 may include a radio-frequency generator with at least one output or output channel, e.g., ten outputs or ten output channels. Each of the outputs may be separately and selectively activated or deactivated by a switch. That is, each switch may be disposed between the signal generator and a respective output. Thus, a generator with ten outputs would include ten switches. These outputs may each be individually coupled to electrodes on an ablation catheter, e.g., the ten electrodes 33 on balloon 80, described in further detail below. Such an electrical connection may be achieved by establishing an electrical path between each output and each electrode. For example, each output may be connected to a corresponding electrode by one or more wires or suitable electrical connectors. Thus, in some embodiments, an electrical path may include at least one wire. In some embodiments, the electrical path may further include an electrical connector and at least a second wire. Thus, electrodes 33 may be selectively activated and deactivated with the switches to receive radiofrequency energy separately from each of the other electrodes.

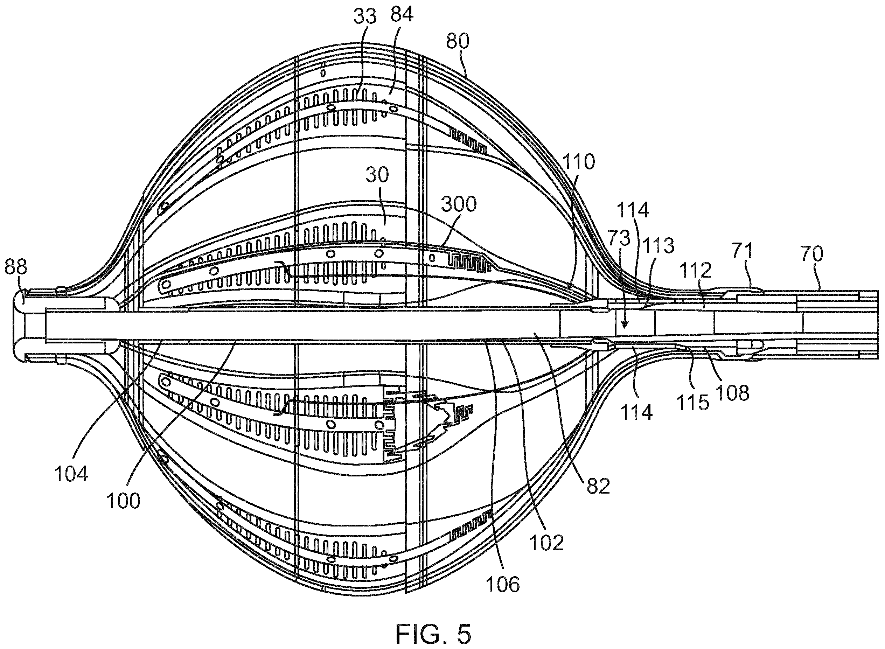

Figure 2 is a schematic perspective view of the diagnostic/therapeutic catheter 24 in in which balloon 80 is in an expanded configuration. Catheter 24 may include a handle 27, a knob 28, a control 37, and irrigation tubing 29. Knob 28 may be used to advance and retract distal collar 88 to change the configuration of balloon 80 between an expanded configuration and a collapsed configuration, as further described below. An irrigation lumen (referred to below as second lumen 112 with reference to Figure 5) passes through catheter 24 and connects tubing 29 to balloon 80, such that an irrigation fluid (e.g., saline) may be introduced or pumped through tubing 29 and ultimately into balloon 80 by a pump connected to a source of the irrigation fluid and irrigation module 53. Control 37 may be used to further manipulate or steer portions of catheter 24, such as by causing a deflection to a distal portion of catheter 24.

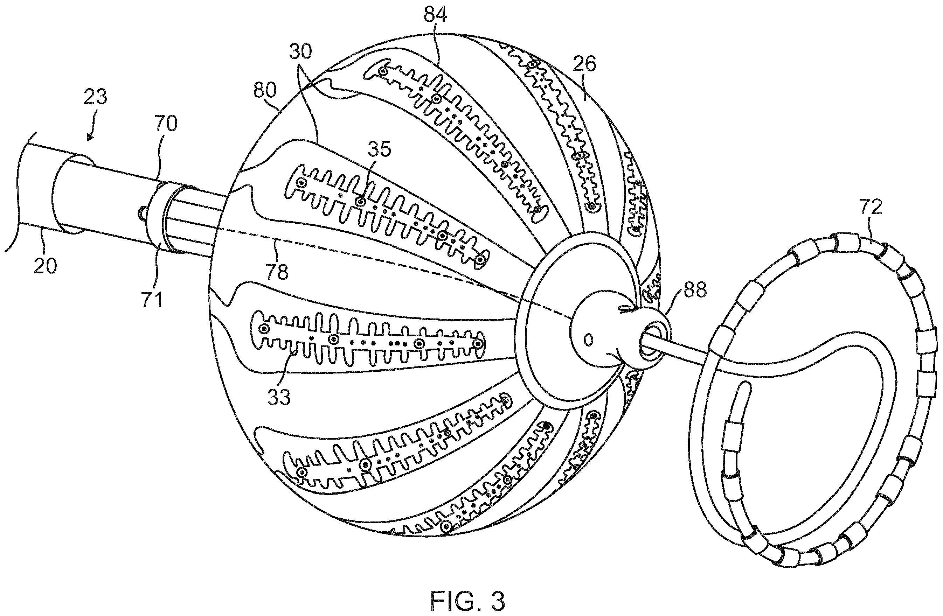

With further reference to Figures 3, 4, and 5 where the diagnostic/therapeutic catheter 24 is used to ablate an ostium 11 of a lumen, such as a pulmonary vein 13, a proximal end (or first end) of balloon 80 may be supported on a distal end of an outer tubular shaft 70, e.g., by being compressed thereon by collar 71, which may also function as a ring electrode. Outer tubular shaft 70 terminates in the balloon at outer shaft opening 73. An inner tubular shaft 82, may be disposed through outer tubular shaft 70 such that it extends into balloon 80 via outer shaft opening 73. A distal end (or second end) of balloon 80 may be attached to a distal end of inner tubular shaft 82 by being compressed thereon by collar 88, which may function as a ring electrode. Another catheter, such as lasso catheter 72, may pass through inner tubular shaft 82 to extend into collar 88. Outer tubular shaft 70, inner tubular shaft 82, and lasso catheter 72 may be disposed with respect to each other in a telescoping relationship. However, the relative movement between outer tubular shaft 70 and inner tubular shaft 82 is limited by the fact that balloon 80 is connected on its proximal or first end to outer tubular shaft 70 and on its distal or second end to inner tubular shaft 82. This relative movement permits for manipulating the shape of balloon 80. Specifically, when the length of inner tubular shaft 82 that extends out of outer tubular shaft 70 is maximized, balloon 80 has an oblong shape, i.e., a collapsed configuration, whereas when the length of inner tubular shaft 82 that extends out of outer tubular shaft 70 is minimized, balloon 80 has a spherical shape, i.e., an expanded configuration, which is the configuration reflected in the figures. This relative movement may be effected by manipulation of knob 28 on handle 27, to move inner tubular shaft 82 relative to outer tubular shaft 70.

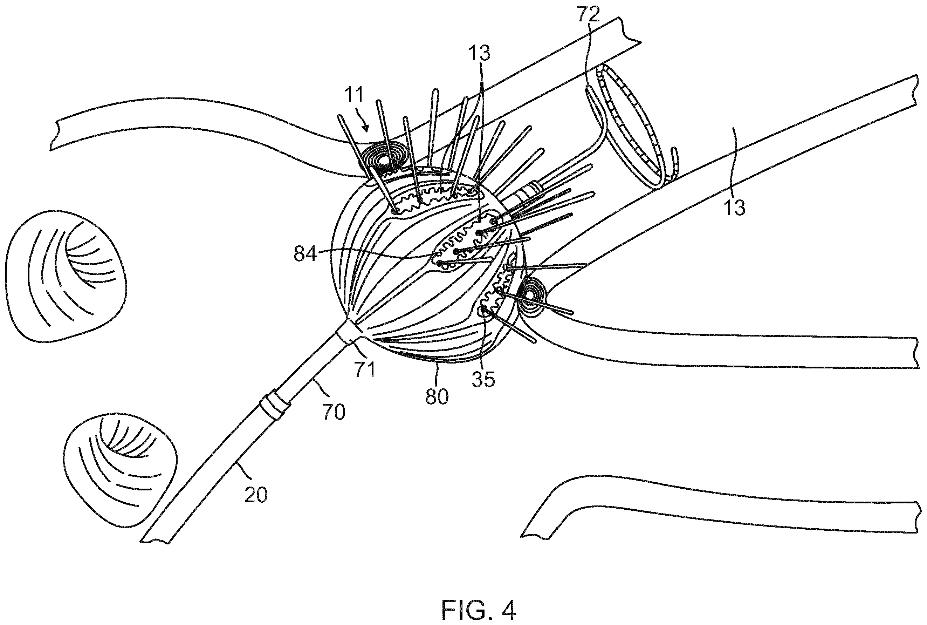

The balloon 80 of the diagnostic/therapeutic catheter 24 has an exterior wall, surface, or membrane 26 of a bio-compatible material, for example, formed from a plastic such as polyethylene terephthalate (PET), polyurethane, or PEBAX^{®}. The outer tubular shaft 70 defines a longitudinal axis 78 of the balloon 80. The balloon 80 is deployed, in a collapsed configuration, via the lumen 23 of the probe 20, and may be expanded after exiting from the distal end as described above. The membrane 26 of the balloon 80 may be formed with irrigation pores or apertures 35 through which fluid (e.g., saline) can exit from the interior of the balloon 80 to outside the balloon for cooling the tissue ablation site at the ostium. While Figure 2 and Figure 4 show fluid exiting the balloon 80 as jet streams, the fluid may exit the balloon with any desired flow rate or pressure, including a rate where the fluid is seeping out of the balloon.

The membrane 26 supports and carries combined electrode and temperature sensing members which are each constructed as a multi-layer flexible circuit electrode assembly 84. The "flex circuit electrode assembly" 84 may have many different geometric configurations. In the illustrated embodiment, the flex circuit electrode assembly 84 has a plurality of radiating substrates or strips 30, upon which are disposed electrodes 33. In the embodiments reflected in the figures, there is one electrode 33 disposed on each of the substrates 30. Thus, for example, where balloon 80 includes ten substrates, it also includes ten electrodes. The substrates 30 are evenly distributed about the distal end 88 and the balloon 80. Each substrate has wider proximal portion that gradually tapers to a narrower distal portion. Further each substrate extends from the first end of balloon 80 at collar 71 to the second end of balloon 80 at collar 88.

### Fluid Management

Applicant has determined that various fluid-management considerations should be accounted for to avoid malfunction and to enable catheter 24 to be used in a clinical setting. For example, due to the telescoping relationship between outer tubular shaft 70 and inner tubular shaft 82, there exists a space between outer tubular shaft 70 and inner tubular shaft 82. Unaccounted for, air might enter this space through handle 27 and enter balloon 80. Further, irrigation fluid in balloon 80 might enter this space via outer shaft opening 73 in balloon 80, where inner tubular shaft 82 extends out of outer tubular shaft 70. Accordingly, Applicant has devised seals that prevent fluids from entering the space between inner tubular shaft 82 and outer tubular shaft 70, but that do not impede the telescopic functionality of inner tubular shaft 82 and outer tubular shaft 70, and that are sufficiently robust to prevent fluid entry for at least five and up to twenty rounds of expanding and collapsing balloon 80 by the telescopic functionality provided by inner tubular shaft 82 and outer tubular shaft 70. Applicant determined that the following characteristics might affect robustness of the seals: 1) friction might cause a seal to bunch up around inner tubular shaft 82, and thus fail, as it is moved back and forth inside outer tubular shaft 70; 2) repeated movement back and forth could cause wear on bearing surfaces of a seal, and this wear might lead to a leak; and 3) the seal should withstand pressures generated inside balloon 80 by flowing irrigation fluid therein. Based on these characteristics and other features of catheter 24, Applicant has devised two different seals, a first or distal seal 100 disposed in balloon 80 to prevent backwash of irrigation fluid, and a second or proximal seal 200 disposed in handle 27 to prevent entry of air.

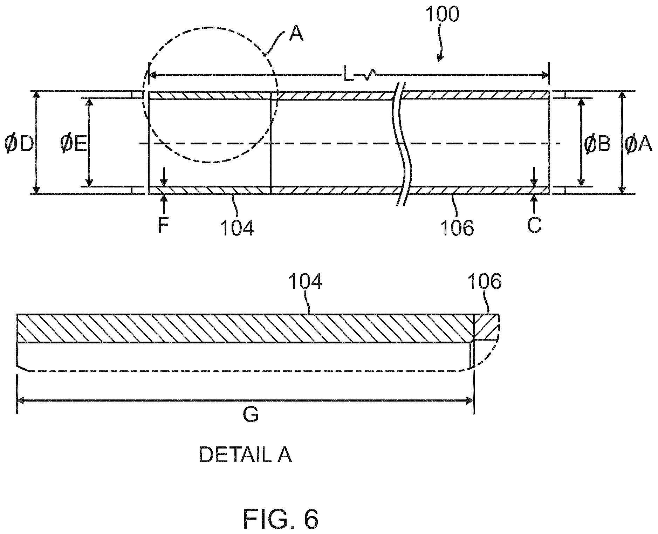

First seal 100 is described with reference to Figures 5 and 6. First seal 100 is disposed about a distal segment 102 of inner tubular shaft 82. Accordingly, first seal 100 may have a tubular configuration, or at least a round inner surface to encompass distal segment 102.

A coupler component 108 may also be provided. The coupler component 108 is also tubular in nature such that it may be attached to the distal portion of outer tubular shaft 70, and such that coupler 108 may be considered a feature of outer tubular shaft 70. As such the proximal or first end of balloon 80 may be attached to outer tubular shaft 70 by or in conjunction with coupler 108 using, e.g., the techniques described above. Accordingly, inner tubular shaft 82 extends into balloon 80, through outer tubular shaft 70 and coupler 108, in region 110 of balloon 80.

Between region 110 and collar 71, i.e., just proximal of region 110, irrigation lumen or second lumen 112 of outer tubular shaft 72 terminates at termination point 113, either at the distal tip of outer tubular shaft 72 or through a sidewall thereof proximate to the distal tip, or a combination thereof. As such, coupler 108 may be provided with at least one port or window 114 through a sidewall 115, with window 114 disposed adjacent to termination point 113 to thus expose termination point 113 to the inside of balloon 80. This enables irrigation fluid pumped through second lumen 112 to flow into balloon 80 via window 114. As shown in Figure 5, there are two instances of window 114.

Irrigation fluid that enters balloon 80 via window 114 pressurizes balloon 80, thus causing its exterior surface 26 to become taut. As such, the pressure in balloon 80 is greater than the pressure in the space between outer tubular shaft 70 and inner tubular shaft 82, giving rise to the need for first seal 100.

First seal 100 may be mated to coupler 108 at the region 110 where inner tubular shaft 82 emerges therefrom. Applicant determined the following characteristics are important to fabricating a robust first seal 100.

First seal 100 preferably includes a distal first-seal portion 104 made from a first material and a proximal first-seal portion 106 made from a second material. The first material and the second material may be different from each other. The first material may comprise: 1) PEBAXTM, a thermoplastic elastomer that consists of polyamide and polyether backbone blocks, manufactured by Arkema; 2) barium sulfate; 3) PROPELLTM low friction compounds manufactured by Foster; or 4) any combination thereof. In a preferred embodiment, the first material may comprise about 76% PEBAX 3533 SA 01 MED, about 20% barium sulfate, and about 4% PROPELL. The second material may comprise VESTAMID^{®}, a polyamide manufactured by Evonik, such as VESTAMID CARE ML21.

With reference to Figure 6, distal first-seal portion 104 has: 1) a pre-assembly wall thickness F of between about 0.00375 inches and about 0.00750 inches, e.g., about 0.00525 inches; 2) a pre-assembly inner diameter E of between about 0.055 inches and about 0.065 inches, e.g., about 0.061 inches. The proximal first-seal portion 106 has: 1) a pre-assembly wall thickness C of between about 0.003 inches and about 0.005 inches, e.g., about 0.00375 inches; and 2) a pre-assembly inner-diameter B of between about 0.0064 inches and about 0.0068 inches, e.g., about 0.0065 inches. As used herein, the term "pre-assembly" indicates characteristics of a component before it has been assembled into the catheter inasmuch as pre-assembly characteristics, particularly pre-assembly dimensions, are used to order materials and determine that they are being provided in compliance with a corresponding product specification.

The above material choices and dimensions enable a friction fit between first seal 100 and inner tubular shaft 82 that does not impede telescopic movement of inner tubular shaft 82 in outer tubular shaft 70, such that distal segment 102 of the inner tubular shaft 82 has an outer diameter greater than or equal to the pre-assembly inner diameter E of the distal first-seal portion. Depending on pressures generated in balloon 80, it may sometimes also be advisable for distal segment 102 of the inner tubular shaft 82 to have an outer diameter greater than or equal to the pre-assembly inner diameter B of the proximal first-seal portion.

To substantially cover distal segment 102, first seal 100 may be between about 10 millimeters and about 25 millimeters long, e.g., about 23 millimeters long. Further, first seal 100 should be shorter than distal segment 102 to avoid interference that might otherwise be caused between, e.g., first seal 100 and collar 88 during assembly of catheter 24. However, the lengths of distal first-seal portion 104 and proximal first-seal portion 106 are different based on the interplay between the dimensions and materials set forth above, and the various properties (e.g., friction) they impart to seal 100 on distal segment 102. Thus, the distal first-seal portion may have a length G of between about 0.09 and about 0.13 inches long, e.g., 0.11 inches long.

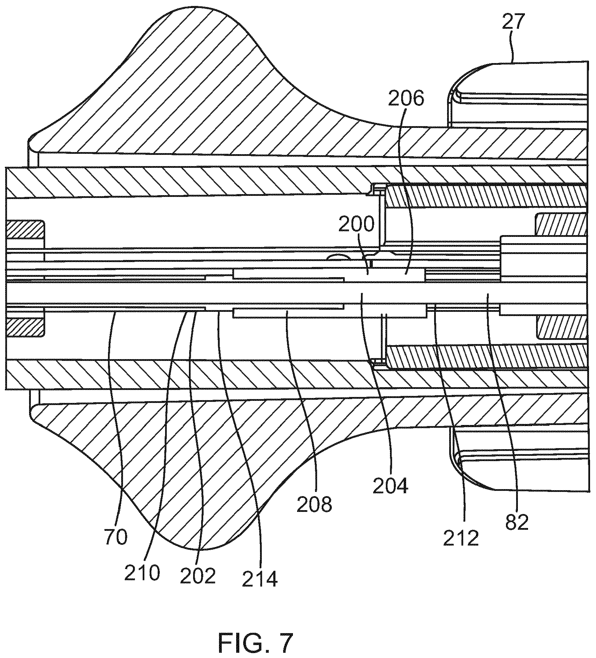

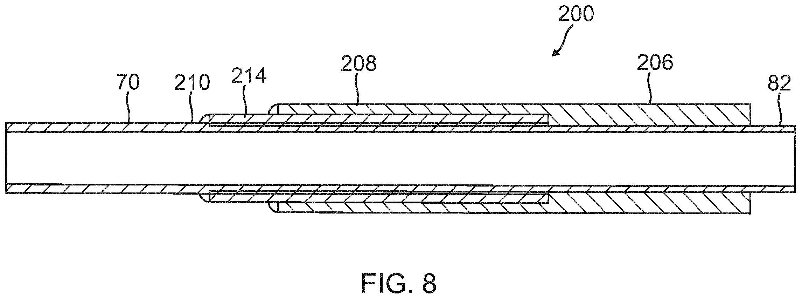

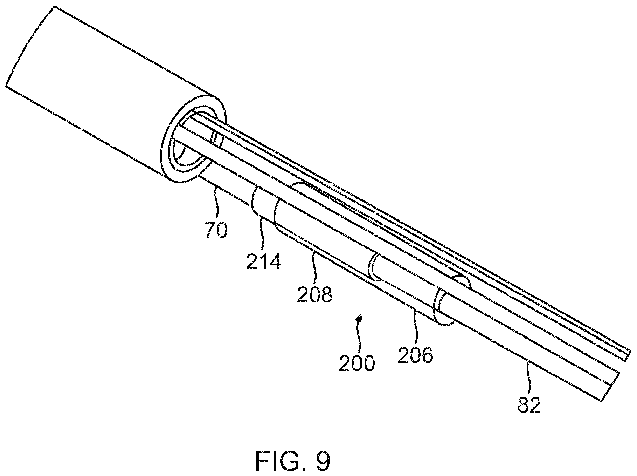

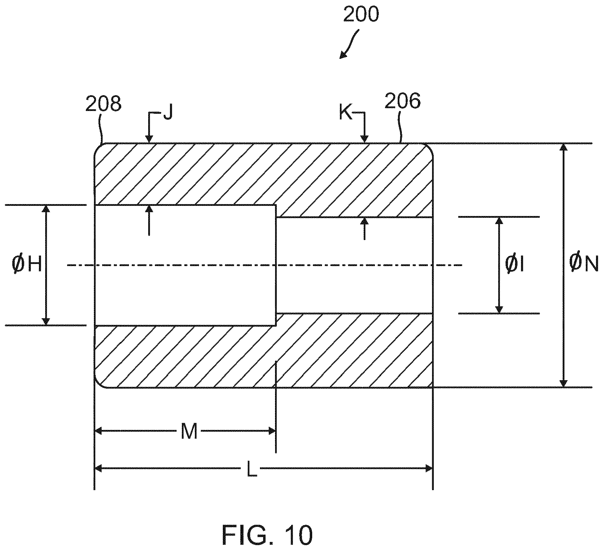

Second seal 200 is now described with reference to Figures 7 to 10. Inner tubular shaft 82 may move telescopically inside outer tubular shaft 70. Further a proximal portion 204 of inner tubular shaft 82 extends out of proximal portion 202 of outer tubular shaft 70. Second seal 200 may be disposed about the location at which proximal portion 204 of inner tubular shaft 82 extends out of proximal portion 202 of outer tubular shaft 70.

Second seal 200 may have a tubular form, including a proximal section 206 and a distal section 208. Distal section 208 may be secured to an outer surface 210 of outer tubular shaft 70. Further, proximal section 206 may be placed into contact with an outer surface 212 of inner tubular shaft 82. The outer diameter N of the proximal section 206 of the second seal 200 is about equal to an outer diameter of the distal section 208 of the second seal 200. Further, a pre-assembly inner diameter I of the proximal section 206 may be less than a pre-assembly inner diameter H of the distal section 208 of the second seal to allow for a seal to be formed about the location where inner tubular shaft 82 enters outer tubular shaft 70 while leaving clearance in distal section 208 for securing distal seal 200 to outer tubular shaft 70. As such, the pre-assembly inner diameter I of the proximal section 206 may be less than or equal to an outer diameter of a segment of the inner tubular shaft 82 disposed through proximal section 206. More specifically: 1) the pre-assembly inner diameter I of proximal section 206 may be between about 0.058 inches and about 0.066 inches, e.g., about 0.062 inches; 2) the pre-assembly inner diameter H of distal section 208 may be between about 0.075 inches and about 0.08 inches, e.g., 0.078 inches; 3) a wall thickness K of proximal section 206 may be between about 0.04 inches and about 0.045 inches, e.g., about 0.0425 inches; and 4) a wall thickness J of distal section 208 may be between about 0.03 inches and about 0.04 inches, e.g., about 0.035 inches.

Second seal 200 may be fabricated from silicone, e.g., Dow Corning's SILASTIC^{®} Q7-6860. A tube 214 comprised of, e.g., a polyimide, may be used to secure outer surface 210 of outer tubular shaft 70 to distal section 208 of seal 200. As such, tube 214 may be disposed at least partially in the distal section of the second seal and about the outer tubular shaft.

### Reinforcement

Through ongoing research and product development efforts concerning the subject matter described above, Applicant has determined that balloon 80 must be able to withstand multiple cycles of being deployed from lumen 23 of probe 20 in the collapsed configuration, expanded to the expanded configuration, returned to the collapsed configuration and withdrawn into lumen 23 of probe 20. The number of cycles may be from about five to about twenty. That is, the connection between substrate 30 and outer surface 26 of balloon 80, as well as the overall integrity of the assembled balloon, must withstand at least about five to about twenty fatigue cycles and any additional frictional stresses experienced during five to twenty deployments from and five to twenty withdrawals into lumen 23. To assist in preventing potential delamination of substrates 30 from outer surface 26, which could arise from repeated fatiguing, Applicant has thus implemented a solution that would prevent, or at least significantly further lower the likelihood of, any such delamination. Applicant further determined that any such solution would need to accommodate for various design constraints, such as: 1) minimizing concomitant safety concerns caused by any solution; 2) minimizing any increase to diameter of the portions of balloon 80 upon which substrates 30 are adhered such that balloon 80 in the collapsed configuration may be readily deployed from and withdrawn into lumen 23 with little or no increase in friction therebetween (or especially, in the extreme, avoiding a need to increase the diameter of lumen 23); 3) minimizing any increase to the stiffness of balloon 80, which could interfere with establishing contact between electrode 30 and tissue during a procedure; 4) not impeding electrical contact between electrodes 33 and tissue during a procedure; and 5) minimizing an increase to the number of assembly steps.

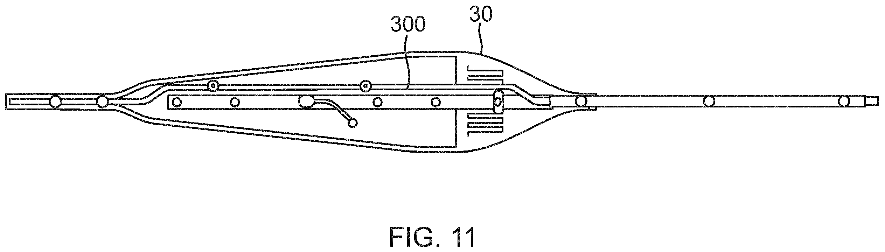

With reference to Figures 5 and 11, a reinforcement component 300 is disclosed that assists in preventing delamination without violating these design constraints. At least one reinforcement component may be disposed along each of substrates 30, extending from the first end of balloon 80, i.e., from collar 71, to the second end of balloon 80, i.e., to collar 88. As such, where balloon 80 includes ten substrates 30, ten reinforcement components 300 may also be provided, one on each of the ten substrates. However, more than one reinforcement component 300 may be provided on each substrate 30. The reinforcement components 300 may be attached to substrates 30 by any suitable method, e.g., with an adhesive.

Preferably, each reinforcement component 300 may have a form of a yarn, and when assembled take the shape of a roughly rectangular cross section having a thickness between about 0.0005 inches and 0.005 inches. The yarn may be fabricated from an ultra-high molecular weight polymer or a liquid-crystal polymer, e.g., VECTRAN^{™}, manufactured by Kuraray. So long as the thickness of the yarn is less than the thickness of electrode 33, it may be disposed on a top surface of substrate 30, i.e., adjacent to electrode 33, such that it would not contact exterior surface 26 of balloon 80. However, if the thickness of the yarn is greater than the thickness of electrode 33, such that the yarn might interfere with the electrode's ability to conform to patient tissue, the yarn should be disposed on a bottom surface of the substrate, such that it would also be disposed directly against exterior surface 26 of balloon 80. Such is the embodiment reflected in Figures 5 and 11.

By virtue of the embodiments illustrated and described herein, Applicant has devised a non-claimed method and variations thereof for using the catheter described herein. In a first variation, the method may include steps of flowing an irrigating fluid through the second lumen and into the balloon, and preventing the irrigation fluid from entering the outer tubular shaft via the distal portion of the outer tubular shaft. In a second variation, the method may include preventing air from entering the outer tubular shaft via the proximal portion of the outer tubular shaft. In a third variation, the method may include steps of extending the balloon out of a probe, expanding the balloon, collapsing the balloon, withdrawing the balloon into the probe, and repeating the steps of extending, expanding, collapsing, and withdrawing between five to twenty times. In a fourth variation, the method may include the steps of the first variation and the second variation. In a fifth variation, the method may include the steps of the first variation and the third variation. In a sixth variation, the method may include the steps of the second variation and the third variation. In a seventh variation, the method may include the steps of the first variation, the second variation, and the third variation.

Any of the examples or embodiments described herein may include various other features in addition to or in lieu of those described above. The teachings, expressions, embodiments, examples, etc., described herein should not be viewed in isolation relative to each other. Various suitable ways in which the teachings herein may be combined should be clear to those skilled in the art in view of the teachings herein.

Having shown and described exemplary embodiments of the subject matter contained herein, further adaptations of the methods and systems described herein may be accomplished by appropriate modifications. Modifications and combinations may fall within the scope of the invention, which is as defined in claim 1. In addition, where methods and steps described above indicate certain events occurring in certain order, it is intended that certain steps do not have to be performed in the order described but in any order as long as the steps allow the embodiments to function for their intended purposes. Therefore, to the extent there are variations of the invention, which are within the scope of the invention defined by the claims, it is the intent that this patent will cover those variations as well. Some such modifications should be apparent to those skilled in the art. For instance, the examples, embodiments, geometrics, materials, dimensions, ratios, steps, and the like discussed above are illustrative. Accordingly, the claims should not be limited to the specific details of structure and operation set forth in the written description and drawings.

## Claims

1. A catheter (24), comprising:
an outer tubular shaft (70) having an outer surface, a first lumen disposed therethrough, and a second lumen disposed therethrough;
an inner tubular shaft (82) having an outer surface disposed in the first lumen of the outer tubular shaft and having a distal portion that extends out of a distal portion of the outer tubular shaft (70), wherein the inner tubular shaft is translatable relative to the outer tubular shaft;
a catheter balloon (80) having a membrane including a first end and a second end, the first end connected to the outer surface of the outer tubular shaft (70) about the distal portion of the outer tubular shaft (70), and the second end connected to the outer surface of the distal portion of the inner tubular shaft, such that a distal segment of the inner tubular shaft is disposed in the balloon, the second lumen for introducing irrigation fluid into the catheter balloon;
a coupler component (108), the coupler component (108) being attached to the distal portion of the outer tubular shaft (70) at a location distal of the first end of the balloon (80) such that the inner tubular shaft (82) extends into the balloon through the coupler component; and
a first distal seal (100) disposed about the distal segment of the inner tubular shaft, the first distal seal comprising a distal seal portion (104) and a proximal seal portion (106), wherein the proximal seal portion (106) mates to the coupler component (108).

2. The catheter (24) of claim 1, in which the first distal seal has a tubular configuration.

3. The catheter (24) of claim 1, in which the second lumen terminates through the outer surface of the outer tubular shaft (70) at a second-lumen opening located in the distal portion of the outer tubular shaft (70) that is in the balloon.

4. The catheter (24) of claim 3, in which the coupler component includes a sidewall and a window disposed through the sidewall that exposes the second-lumen opening.

5. The catheter (24) of claim 3, in which the distal seal portion (104) comprises a first material and the proximal seal portion (106) comprises a second material.

6. The catheter (24) of claim 3, in which the distal seal portion (104) has a pre-assembly wall thickness of between 0.009525 cm and 0.01905 cm (about 0.00375 inches and 0.00750 inches), preferably in which the pre-assembly wall thickness of the distal seal portion (104) is 0.01334 cm (0.00525 inches).

7. The catheter (24) of claim 6, in which the distal seal portion (104) has a pre-assembly inner diameter of between 0.140 cm and 0.165 cm (0.055 inches and 0.065 inches), preferably in which the pre-assembly inner diameter of the distal seal portion (104) is 0.155 cm (0.061 inches).

8. The catheter (24) of claim 7, in which the proximal seal portion (106) has a pre-assembly wall thickness of between 0.0076 cm and 0.013 cm (0.003 inches and 0.005 inches), preferably in which the pre-assembly wall thickness of the proximal seal portion (106) is 0.009525 cm (0.00375 inches).

9. The catheter (24) of claim 8, in which the proximal seal portion (106) has a pre-assembly inner-diameter of between 0.0163 cm and 0.0173 cm (0.0064 inches and 0.0068 inches), preferably in which the pre-assembly inner diameter of the proximal seal portion (106) is 0.0165 cm (0.0065 inches).

10. The catheter (24) of claim 9, in which the distal segment of the inner tubular shaft (82) has an outer diameter greater than or equal to the pre-assembly inner diameter of the distal seal portion (104), preferably greater than or equal to the pre-assembly inner diameter of the proximal seal portion (106).

11. The catheter (24) of claim 10, in which the first distal seal (100) is between about 10 millimeters and about 25 millimeters long, preferably in which the first distal seal (100) is about 23 millimeters long.

12. The catheter (24) of claim 10, wherein the distal segment of the inner tubular shaft (82) has an outer diameter greater than or equal to the pre-assembly inner diameter of the proximal seal portion (106), and in which the first distal seal (100) is shorter than the distal segment of the inner tubular shaft (82).

13. The catheter (24) of claim 12, in which the distal seal portion (104) is between 0.29 cm and 0.330 cm (0.09 inches and 0.13 inches)long, preferably in which the distal seal portion (104) is 0.279 cm (0.11 inches) long.

14. The catheter (24) of claim 13, in which the distal seal portion (104) is disposed about the inner tubular shaft (82) in a friction-fit relationship.

15. The catheter (24) of claim 14, in which the proximal seal portion (106) is disposed about the inner tubular shaft (82) in a friction-fit relationship.

## Patentansprüche

1. Katheter (24), umfassend:
einen äußeren Rohrschaft (70), der eine äußere Oberfläche, ein erstes Lumen, das dahindurch angeordnet ist, und ein zweites Lumen, das dahindurch angeordnet ist, aufweist;
einen inneren Rohrschaft (82), der eine äußere Oberfläche aufweist, die in dem ersten Lumen des äußeren Rohrschafts angeordnet ist und einen distalen Abschnitt, der sich aus einem distalen Abschnitt des äußeren Rohrschafts (70) heraus erstreckt, aufweist, wobei der innere Rohrschaft relativ zum äußeren Rohrschaft verlagerungsfähig ist;
einen Katheterballon (80), der eine Membran, einschließlich eines ersten Endes und eines zweiten Endes aufweist, wobei das erste Ende mit der äußeren Oberfläche des äußeren Rohrschafts (70) um den distalen Abschnitt des äußeren Rohrschafts (70) verbunden ist und das zweite Ende mit der äußeren Oberfläche des distalen Abschnitts des inneren Rohrschafts verbunden ist, derart, dass ein distales Segment des inneren Rohrschafts in dem Ballon, dem zweiten Lumen zum Einbringen von Spülfluid in den Katheterballon angeordnet ist;
eine Kopplerkomponente (108), wobei die Kopplerkomponente (108) an dem distalen Abschnitt des äußeren Rohrschafts (70) an einer Stelle distal des ersten Endes des Ballons (80) befestigt ist, derart, dass sich der innere Rohrschaft (82) durch die Kopplerkomponente in den Ballon erstreckt; und
eine erste distale Dichtung (100), die um das distale Segment des inneren Rohrschafts angeordnet ist, die erste distale Dichtung umfassend einen distalen Dichtungsabschnitt (104) und einen proximalen Dichtungsabschnitt (106), wobei der proximale Dichtungsabschnitt (106) mit der Kopplerkomponente (108) zusammenpasst.

2. Katheter (24) nach Anspruch 1, wobei die erste distale Dichtung eine Rohrkonfiguration aufweist.

3. Katheter (24) nach Anspruch 1, wobei das zweite Lumen durch die äußere Oberfläche des äußeren Rohrschafts (70) an einer Öffnung des zweiten Lumens, die sich in dem distalen Abschnitt des äußeren Rohrschafts (70) befindet, endet, der in dem Ballon ist.

4. Katheter (24) nach Anspruch 3, wobei die Kopplerkomponente eine Seitenwand und ein Fenster, das durch die Seitenwand angeordnet ist, einschließt, das die Öffnung des zweiten Lumens freigibt.

5. Katheter (24) nach Anspruch 3, wobei der distale Dichtungsabschnitt (104) ein erstes Material und der proximale Dichtungsabschnitt (106) ein zweitens Material umfasst.

6. Katheter (24) nach Anspruch 3, wobei der distale Dichtungsabschnitt (104) vor einem Zusammenbau eine Wanddicke von zwischen 0,009525 cm und 0,01905 cm (etwa 0,00375 Zoll und 0,00750 Zoll) aufweist, vorzugsweise wobei die Wanddicke des distalen Dichtungsabschnitts (104) vor dem Zusammenbau 0,01334 cm (0,00525 Zoll) beträgt.

7. Katheter (24) nach Anspruch 6, wobei der distale Dichtungsabschnitt (104) vor einem Zusammenbau einen inneren Durchmesser von zwischen 0,140 cm und 0,165 cm (0,055 Zoll und 0,065 Zoll) aufweist, vorzugsweise wobei der innere Durchmesser des distalen Dichtungsabschnitts (104) vor dem Zusammenbau 0,155 cm (0,061 Zoll) beträgt.

8. Katheter (24) nach Anspruch 7, wobei der proximale Dichtungsabschnitt (106) vor einem Zusammenbau eine Wanddicke von zwischen 0,0076 cm und 0,013 cm (0,003 Zoll und 0,005 Zoll) aufweist, vorzugsweise wobei die Wanddicke des proximalen Dichtungsabschnitts (106) vor dem Zusammenbau 0,009525 cm (0,00375 Zoll) beträgt.

9. Katheter (24) nach Anspruch 8, wobei der proximale Dichtungsabschnitt (106) vor einem Zusammenbau einen inneren Durchmesser von zwischen 0,0163 cm und 0,0173 cm (0,0064 Zoll und 0,0068 Zoll) aufweist, vorzugsweise wobei der innere Durchmesser des proximalen Dichtungsabschnitts (106) vor dem Zusammenbau 0,0165 cm (0,0065 Zoll) beträgt.

10. Katheter (24) nach Anspruch 9, wobei das distale Segment des inneren Rohrschafts (82) einen äußeren Durchmesser, der größer als oder gleich dem inneren Durchmesser des distalen Dichtungsabschnitts (104) vor dem Zusammenbau, vorzugsweise größer als oder gleich dem inneren Durchmesser des proximalen Dichtungsabschnitts (106) vor dem Zusammenbau, ist, aufweist.

11. Katheter (24) nach Anspruch 10, wobei die erste distale Dichtung (100) zwischen etwa 10 Millimeter und etwa 25 Millimeter lang ist, vorzugsweise wobei die erste distale Dichtung (100) etwa 23 Millimeter lang ist.

12. Katheter (24) nach Anspruch 10, wobei das distale Segment des inneren Rohrschafts (82) einen äußeren Durchmesser, der größer als oder gleich dem inneren Durchmesser des proximalen Dichtungsabschnitts (106) vor dem Zusammenbau ist, aufweist, und wobei die erste distale Dichtung (100) kürzer als das distale Segment des inneren Rohrschafts (82) ist.

13. Katheter (24) nach Anspruch 12, wobei der distale Dichtungsabschnitt (104) zwischen 0,29 cm und 0,330 cm (0,09 Zoll und 0,13 Zoll) lang ist, vorzugsweise wobei der distale Dichtungsabschnitt (104) 0,279 cm (0,11 Zoll) lang ist.

14. Katheter (24) nach Anspruch 13, wobei der distale Dichtungsabschnitt (104) in einer Reibpassung um den inneren Rohrschaft (82) angeordnet ist.

15. Katheter (24) nach Anspruch 14, wobei der proximale Dichtungsabschnitt (106) in einer Reibpassung um den inneren Rohrschaft (82) angeordnet ist.

## Revendications

1. Cathéter (24) comprenant :
une tige tubulaire externe (70) ayant une surface externe, une première lumière disposée à travers celle-ci, et une seconde lumière disposée à travers celle-ci ;
une tige tubulaire interne (82) ayant une surface externe disposée dans la première lumière de la tige tubulaire externe et ayant une partie distale qui s'étend hors d'une partie distale de la tige tubulaire externe (70), dans lequel la tige tubulaire interne peut être translatée par rapport à la tige tubulaire externe ;
un ballonnet de cathéter (80) ayant une membrane comportant une première extrémité et une seconde extrémité, la première extrémité étant reliée à la surface externe de la tige tubulaire externe (70) autour de la partie distale de la tige tubulaire externe (70), et la seconde extrémité étant reliée à la surface externe de la partie distale de la tige tubulaire interne, de telle sorte qu'un segment distal de la tige tubulaire interne est disposé dans le ballonnet, la seconde lumière permettant d'introduire un fluide d'irrigation dans le ballonnet de cathéter ;
un composant d'accouplement (108), le composant d'accouplement (108) étant fixé à la partie distale de la tige tubulaire externe (70) au niveau d'un emplacement distal de la première extrémité du ballonnet (80) de telle sorte que la tige tubulaire interne (82) s'étend dans le ballonnet à travers le composant d'accouplement ; et
un premier joint d'étanchéité distal (100) disposé autour du segment distal de la tige tubulaire interne, le premier joint d'étanchéité distal comprenant une partie de joint d'étanchéité distale (104) et une partie de joint d'étanchéité proximale (106), dans lequel la partie de joint d'étanchéité proximale (106) s'apparie au composant d'accouplement (108).

2. Cathéter (24) selon la revendication 1, dans lequel le premier joint d'étanchéité distal a une configuration tubulaire.

3. Cathéter (24) selon la revendication 1, dans lequel la seconde lumière se termine à travers la surface externe de la tige tubulaire externe (70) au niveau d'une ouverture de seconde lumière située dans la partie distale de la tige tubulaire externe (70) qui se trouve dans le ballonnet.

4. Cathéter (24) selon la revendication 3, dans lequel le composant d'accouplement comporte une paroi latérale et une fenêtre disposée à travers la paroi latérale qui expose l'ouverture de seconde lumière.

5. Cathéter (24) selon la revendication 3, dans lequel la partie de joint d'étanchéité distale (104) comprend un premier matériau et la partie de joint d'étanchéité proximale (106) comprend un second matériau.

6. Cathéter (24) selon la revendication 3, dans lequel la partie de joint d'étanchéité distale (104) a une épaisseur de paroi avant assemblage comprise entre 0,009525 cm et 0,01905 cm (environ 0,00375 pouces et 0,00750 pouces), de préférence dans lequel l'épaisseur de paroi avant assemblage de la partie de joint d'étanchéité distale (104) est de 0,01334 cm (0,00525 pouces).

7. Cathéter (24) selon la revendication 6, dans lequel la partie de joint d'étanchéité distale (104) a un diamètre interne avant assemblage compris entre 0,140 cm et 0,165 cm (0,055 pouces et 0,065 pouces), de préférence dans lequel le diamètre interne avant assemblage de la partie de joint d'étanchéité distale (104) est de 0,155 cm (0,061 pouces).

8. Cathéter (24) selon la revendication 7, dans lequel la partie de joint d'étanchéité proximale (106) a une épaisseur de paroi avant assemblage comprise entre 0,0076 cm et 0,013 cm (0,003 pouces et 0,005 pouces), de préférence dans lequel l'épaisseur de paroi avant assemblage de la partie de joint d'étanchéité proximale (106) est de 0,009525 cm (0,00375 pouces).

9. Cathéter (24) selon la revendication 8, dans lequel la partie de joint d'étanchéité proximale (106) a un diamètre intérieur de pré-assemblage compris entre 0,0163 cm et 0,0173 cm (0,0064 pouces et 0,0068 pouces), de préférence dans lequel le diamètre interne de pré-assemblage de la partie de joint d'étanchéité proximale (106) est de 0,0165 cm (0,0065 pouces).

10. Cathéter (24) selon la revendication 9, dans lequel le segment distal de la tige tubulaire interne (82) a un diamètre externe supérieur ou égal au diamètre interne avant assemblage de la partie de joint d'étanchéité distale (104), de préférence supérieur ou égal au diamètre interne avant assemblage de la partie de joint d'étanchéité proximale (106).

11. Cathéter (24) selon la revendication 10, dans lequel le premier joint d'étanchéité distal (100) a une longueur comprise entre environ 10 millimètres et environ 25 millimètres, de préférence dans lequel le premier joint d'étanchéité distal (100) a une longueur d'environ 23 millimètres.

12. Cathéter (24) selon la revendication 10, dans lequel le segment distal de la tige tubulaire interne (82) a un diamètre externe supérieur ou égal au diamètre interne avant assemblage de la partie de joint d'étanchéité proximale (106), et dans lequel le premier joint d'étanchéité distal (100) est plus court que le segment distal de la tige tubulaire interne (82).

13. Cathéter (24) selon la revendication 12, dans lequel la partie de joint d'étanchéité distale (104) a une longueur comprise entre 0,29 cm et 0,330 cm (0,09 pouces et 0,13 pouces), de préférence dans lequel la partie de joint d'étanchéité distale (104) a une longueur de 0,279 cm (0,11 pouces).

14. Cathéter (24) selon la revendication 13, dans lequel la partie de joint d'étanchéité distale (104) est disposée autour de la tige tubulaire interne (82) dans une relation d'ajustement par friction.

15. Cathéter (24) selon la revendication 14, dans lequel la partie de joint d'étanchéité proximale (106) est disposée autour de la tige tubulaire interne (82) dans une relation d'ajustement par friction.
